# EUROPÄISCHE PATENTSCHRIFT

(11) **EP 2 114 847 B1**
(45) Veröffentlichungstag und Bekanntmachung des Hinweises auf die Patenterteilung: **22.03.2017**
(21) Anmeldenummer: 08715804.4
(22) Anmeldetag: 16.02.2008
(51) Int. Cl.: C07C 45/46, C07C 49/84

(54) **VERFAHREN ZUR HERSTELLUNG VON 2,4-DIHYDROXYPHENYL-BENZYL-KETONEN**
METHOD FOR THE PRODUCTION OF 2,4-DIHYDROXYPHENYL-BENZYL KETONES
PROCÉDÉ DE PRODUCTION DE 2,4-DIHYDROXYPHÉNYL-BENZYL-CÉTONES

(30) Priorität: 26.02.2007 EP 07003860
(43) Veröffentlichungstag der Anmeldung: 11.11.2009
(73) Patentinhaber: Plant Health Care, Inc., Raleigh NC 27608 (US)
(72) Erfinder: PAZENOK, Sergii, 42699 Solingen (DE); LUI, Norbert, 51519 Odenthal (DE)
(74) Vertreter: Grünecker Patent- und Rechtsanwälte PartG mbB
(86) Internationale Anmeldenummer: PCT/EP2008/001203
(87) Internationale Veröffentlichungsnummer: WO 2008/104297

(56) Entgegenhaltungen:
- EP-A1- 0 295 883
- DE-A1- 2 616 986
- US-A- 4 225 619
- US-A- 4 269 965
- US-A- 5 235 109
- US-A- 5 981 775
- US-A1- 2006 129 002

## Beschreibung

Die Erfindung betrifft ein Verfahren zur Herstellung von 2,4-Dihydroxyphenyl-4-methoxybenzylketonen der Formel (I) durch Friedel Crafts Acylierung in Fluorwasserstoff (HF). 2,4-Dihydroxyphenyl-4-methoxybenzyl-ketone der allgemeinen Formel (I) sind wichtige Synthesebausteine zur Herstellung von Isoflavonen, wie beispielsweise dem Formononetin oder Genistein, Daidzein und Coumestrol (allgemeine Formel (IV)).

2,4-Dihydroxyphenyl-4-methoxybenzyl-ketone wurden beispielsweise mit sehr mäßiger Ausbeute durch die zeitaufwendige Houben-Hoesch Reaktion ausgehend von Phenylessigsäurenitril, Resorcinol und Chlorwasserstoff erhalten (W. Baker et al., J.Chem.Soc 1929, 2902).

Ferner wurde die Umsetzung von Resorcinol und Phenylessigsäureanhydrid oder der freien Säure in Gegenwart von Borontrifluoridetherat mit einer Ausbeute von 67% beschrieben (S.Mohaty et al., Current Science, May 20, 1988, vol. 57, N. 10 und US 5,981,775).

Des Weiteren wurde eine Synthese von Deoxybenzoin aus Resorcinol und 4-Methoxyphenylessigsäure in Gegenwart von 40 Moläquivalenten eines BF₃/Et₂O Komplexes beschrieben (T.A. Hase in J.Chem Soc. Perkin Trans. 1, 1991, 3005).

Außerdem wurden die Synthese von 2-Phenylacetophenonderivaten aus Phenol und Phenylessigsäure unter Verwendung von Dicyclohexylcarbodiimid (DCC) und 4-Dimethylaminopyridin (DMAP) beschrieben (WO 2005/054169).

Es ist weiterhin bekannt, dass Benzoine mittels Friedel Crafts Acylierung in Gegenwart von AlCl₃ erzeugt werden können (Indian J.Chem. vol. 6, 1968, p. 482).

Weiterhin ist bekannt, dass Polyhydroxybenzoine durch eine Mikrowellensynthese ionischer Flüssigkeiten in Gegenwart von Bis(trifluoromethyl)sulfonyl-amin oder BF₃/OEt₂ zugänglich sind (Tetrahedron Letters, 47, 2006, 8375).

All diese Methoden weisen eine Reihe von Nachteilen auf. Diese sind eine niedrige Ausbeute, lange Reaktionszeiten oder die Verwendung teurer Reagenzien wie DCC und DMAP. Die Anwendung von Katalysatoren wie BF₃ und AlCl₃ erzeugt große Mengen aufwändig zu entsorgender Abwässer.

Der BF₃/Et₂O Komplex ist für eine großtechnische Synthese sogar praktisch ungeeignet, da er einen sehr niedrigen Flammpunkt besitzt.

US 5,235,109 offenbart die Herstellung von Ketonen unter Verwendung von wasserfreiem Aluminiumchlorid in Dichlorethan.

Es wurde nun gefunden, dass die Synthese von 2,4-Dihydroxyphenyl-4-methoxybenzyl Ketonen der Formel (I), 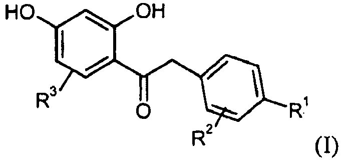 in welchen
- R¹ und R²: für Wasserstoff, Chlor, Fluor, Brom, Iod, CF₃, Methyl, Methoxy, gegebenenfalls substituiertes Alkoxy, -OCF₃, -C(CH₃)₃, -CH(CH₃)₂ stehen,
- R³: für Wasserstoff, Cl, F, Br oder gegebenenfalls substituiertes Alkyl, gegebenenfalls substituiertes Alkoxy, -C(CH3)₃ stehen und
- X: für Hydroxy, F, Cl oder gegebenenfalls substituiertes Alkoxy oder für Br steht,
durch Umsetzung von Phenylessigsäurederivaten der Formel (II) mit Phenolen der Formel (III) in flüssigem Fluorwasserstoff (HF) mit hoher Ausbeute und in hoher Reinheit möglich ist. 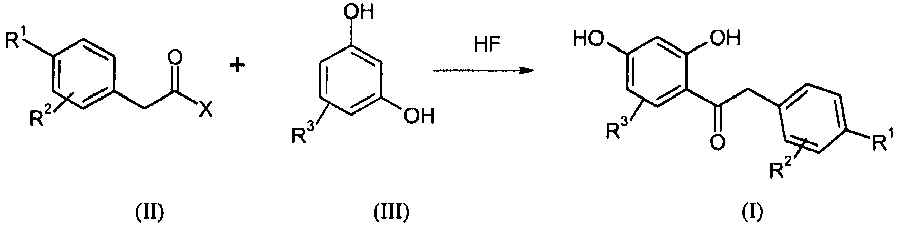 wobei kein weiteres Verdünnungsmittel verwendet wird, die Reaktion drucklos unter Eigendruck stattfindet und die Reaktionstemperatur zwischen 10°C und 50°C liegt.

Ein weiterer Vorteil des Verfahrens besteht darin, dass HF mit einem Siedepunkt von 20°C leicht vom Produkt durch Destillation abgetrennt und daher vollständig zurückgeführt werden kann. Außerdem ist HF ein sehr preiswerter Rohstoff und wird großtechnisch im Tausend-Tonnen-Maßstab hergestellt und eingesetzt.
- R¹ und R²: stehen bevorzugt für Wasserstoff, Methyl, Methoxy, C₁-C₆-Alkoxy, -OCF₃, -C(CH₃)₃, -CH(CH₃)₂, oder Chlor, Fluor, Brom, Iod, CF₃.
- R¹ und R²: stehen besonders bevorzugt für Wasserstoff, Methyl, Methoxy, C₁-C₄-Alkoxy, -C(CH₃)₃, -CH(CH₃)₂, oder Chlor, Fluor, Brom, Iod, -CF₃.
- R¹: steht ganz besonders bevorzugt für Methoxy.
- R²: steht ganz besonders bevorzugt für Wasserstoff.
- R³: steht bevorzugt für Wasserstoff, C₁-C₆-Alkyl, Chlor, Fluor, Brom, C₁-C₆-Alkoxy, -C(CH₃)₃.
- R³: steht besonders bevorzugt für Wasserstoff, C₁-C₄-Alkyl, Cl, F, Br, C₁-C₄-Alkoxy, -C(CH₃)₃.
- R³: steht ganz besonders bevorzugt für Wasserstoff.
- X: steht bevorzugt für Hydroxy, Fluor, Chlor, C₁-C₆-Alkoxy und besonders bevorzugt für Hydroxy, Fluor, Chlor, C₁-C₄-Alkoxy.
- X: steht ferner bevorzugt für Brom.

Mögliche Substituenten für Alkyl und Alkoxy, sind: Fluor, Chlor, Brom, Iod, NO₂, CN, SCN, NCO. Die Reaktion kann gegebenenfalls durch den Zusatz weiterer Katalysatoren beschleunigt werden. Beispielsweise können Katalysatoren wie z.B. Lewis Säuren wie BF₃, SbF₅, PF₅, BiF₃, AsF₃, AlCl₃, SbCl₅, TiCl₄, NbCl₅, SnCl₄, SiCl₄ und InCl₃ eingesetzt werden. Bevorzugte Katalysatoren sind: BF₃, SbCl₅, AlCl₃, SiCl₄, PF₅. Besonders bevorzugte Katalysatoren sind BF₃, AlCl₃, SbCl₃.

Die Reaktionstemperaturen können bei der Durchführung des erfindungsgemäßen Verfahrens in einem größeren Bereich variiert werden. Im Allgemeinen liegen die Temperaturen zwischen 10°C und 50°C. Besonders bevorzugt sind Reaktionstemperaturen zwischen 20°C und 40°C.

Die Molverhältnisse des Fluorwasserstoffs zum Phenol der Formel (III) sind in einem weiten Bereich variierbar. Allgemein wird das erfindungsgemäße Verfahren mit Molverhältnissen von Fluorwasserstoff zum Phenol der Formel (III) zwischen 1:1 bis 100:1 durchgeführt. Bevorzugt sind Molverhältnisse von 50:1 bis 10:1.

Erfindungsgemäß wird das Verfahren ohne weitere Verdünnungsmittel durchgeführt.

Die Reaktion wird drucklos unter Eigendruck durchgeführt.

Die Verbindungen der allgemeinen Formel (I) können gemäß folgendem Schema zu Verbindungen der allgemeinen Formel (IV) umgesetzt werden.

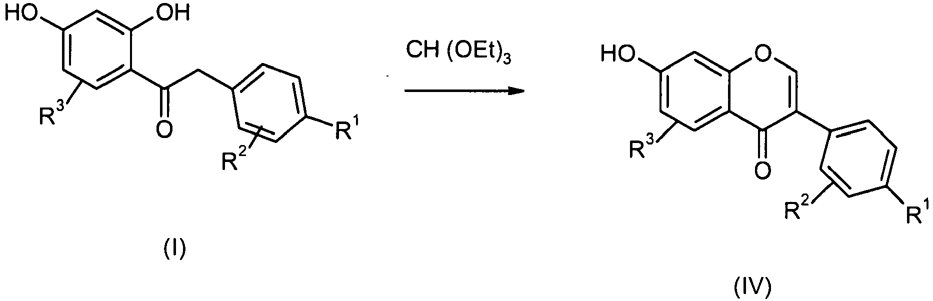

Für die Reaktion eignen sich neben CH(OEt)₃ allgemeine Verbindungen, die neben einer CH-Struktureinheit über die nucleophile Abgangsgruppen verfügen (Pivovarenko et al., Klim. Pirod. Soed. (Ukraine) 5 (1989), 639-643).

### Herstellungsbeispiele

### Herstellung von 1-(2,4-dihydroxyphenyl)-2-(4-methoxyphenyl)-ethanon

### Bespiel 1

4-Methoxyphenylessigsäure (83.9 g), Resorcinol (55 g) und Fluorwasserstoff (450 g) werden bei -10°C im Autoklav vorgelegt und das Gemisch 12 h bei 20°C gerührt. Anschließend wird der Fluorwasserstoff bei 40°C abgedampft und der Niederschlag mit Wasser gewaschen und getrocknet.

Man erhält 123 g (91 % der Theorie) des Produktes mit einer Reinheit von 96 % und einem Schmelzpunkt (Smp) 160-162°C.

### Beispiel 2

4-Methoxyphenylessigsäurechlorid (93 g), Resorcinol (55 g) und Fluorwasserstoff (450 g) werden bei -10°C im Autoklav vorgelegt und das Gemisch 12 h bei 20°C gerührt. Anschließend wird der Fluorwasserstoff bei 40°C abgedampft und der Niederschlag mit Wasser gewaschen und getrocknet.

Man erhält 125 g (92 % der Theorie) des Produktes mit einer Reinheit von 96 %.

### Beispiel 3

4-Methoxyphenylessigsäure (83.9 g), Resorcinol (55 g) und Fluorwasserstoff (300 g) werden bei - 10°C im Autoklav vorgelegt und das Gemisch 12 h bei 20°C gerührt. Anschließend wird der Fluorwasserstoff bei 40°C abgedampft und der Niederschlag mit Wasser gewaschen und getrocknet.

Man erhält 120 g (89 % der Theorie) des Produktes mit einer Reinheit von 93 %.

## Patentansprüche

1. Verfahren zur Herstellung von 2,4-Dihydroxyphenylbenzyl-ketonen der Formel (I), 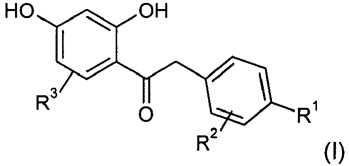
in welchen R¹ und R² für Wasserstoff, Chlor, Fluor, Brom, Iod, CF₃, Methyl gegebenenfalls substituiertes Alkoxy, -OCF₃, -C(CH₃)₃, -CH(CH₃)₂ stehen,
R³ für Wasserstoff, Cl, F, Br, gegebenenfalls substituiertes Alkyl, gegebenenfalls substituiertes Alkoxy, C(CH₃)₃ steht und
X für Hydroxy, F, Cl, Br, gegebenenfalls substituiertes Alkoxy steht,
durch Umsetzung von Phenylessigsäurederivaten der Formel (II) mit Phenolen der Formel (III) in Fluorwasserstoff, 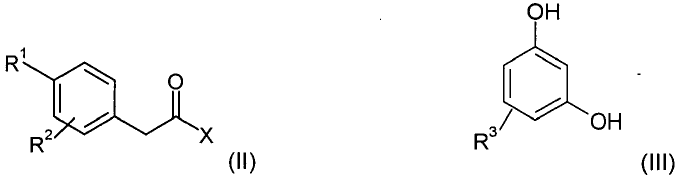 wobei kein weiteres Verdünnungsmittel verwendet wird, die Reaktion drucklos unter Eigendruck stattfindet und die Reaktionstemperatur zwischen 10°C und 50°C liegt.

2. Verfahren gemäß Anspruch 1, wobei
R¹ und R² für Wasserstoff, Chlor, Fluor, Brom, Iod, CF₃, Methyl, C₁-C₆-Alkoxy, -OCF₃, -C(CH₃)₃, CH(CH₃)₂ stehen,
R³ für Wasserstoff, Cl, F, Br, C₁-C₆-Alkyl, C₁-C₆-Alkoxy, -C(CH₃)₃, steht und
X für Hydroxy, F, Cl, Br, C₁-C₆-Alkoxy steht.

3. Verfahren gemäß Anspruch 1, wobei
R¹ und R² für Wasserstoff, Chlor, Fluor, Brom, Iod, CF₃, Methyl, C₁-C₄-Alkoxy, -C(CH₃)₃, -CH(CH₃)₂ stehen,
R³ für Wasserstoff, Cl, F, Br, C₁-C₆-Alkyl, C₁-C₄-Alkoxy, C(CH₃)₃ steht und
X für Hydroxy, F, Cl, C₁-C₄-Alkoxy steht.

4. Verfahren gemäß Anspruch 1 zur Herstellung von 1-(2,4-Dihydroxyphenyl)-2-(4-methoxyphenyl)-ethanon, **dadurch gekennzeichnet, dass** 4-Methoxyphenylessigsäure oder 4-Methoxyphenylessigsäurechlorid mit Resorcinol und mit Fluorwasserstoff ohne weitere Verdünnungsmittel umgesetzt wird.

5. Verfahren gemäß Anspruch 1, **dadurch gekennzeichnet, dass** das Molverhältnis von Fluorwasserstoff zum Phenol der Formel (III) im Bereich von 50:1 bis 10:1 liegt.

6. Verfahren gemäß Anspruch 1, **dadurch gekennzeichnet, dass** die Reaktion in Anwesenheit einer Lewis-Säure abläuft.

7. Verfahren zur Herstellung von 1-(2,4-Dihydroxyphenyl)-2-(4-methoxyphenyl)-ethanon, **dadurch gekennzeichnet, dass** 4-Methoxyphenylessigsäure oder 4-Methoxyphenylessigsäurechlorid mit Resorcinol und mit Fluorwasserstoff, bei einem Molverhältnis von Fluorwasserstoff zu Resorcinol im Bereich von 50:1 bis 10:1 und bei einer Temperatur in einem Bereich von 0°C bis 40°C, umgesetzt wird, wobei kein weiteres Verdünnungsmittel verwendet wird und die Reaktion drucklos unter Eigendruck stattfindet.

8. Verfahren zur Herstellung von Verbindungen der allgemeinen Formel (IV), indem man die Verbindungen der allgemeinen Formeln (II) und (III) nach dem Verfahren gemäß Anspruch 1 zu Verbindungen 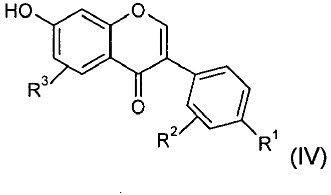 der allgemeinen Formel (I) umsetzt und diese anschließend zu Flavonen der allgemeinen Formel (IV) cyclisiert.

## Claims

1. A method for preparing 2,4-dihydroxyphenyl-benzyl-ketones of the formula (I), 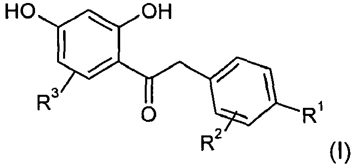 wherein
R¹ and R² represent hydrogen, chlorine, fluorine, bromine, iodine, CF₃, methyl, methoxy, optionally substituted alkoxy, -OCF₃, -C(CH₃)₃, -CH(CH₃)₂;
R³ represent hydrogen, Cl, F, Br, or optionally substituted alkyl, optionally substituted alkoxy, -C(CH₃)₃; and
X represent hydroxyl, F, Cl, Br, or optionally substituted alkoxy;
by reacting phenylacetic acid derivatives of the formula (II) with phenols of the formula (III) in hydrogen fluoride (HF), 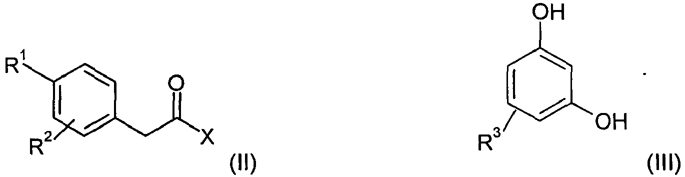 wherein no further diluent is used, and wherein the reaction is performed at ambient temperature under autogenous pressure, and the reaction temperature is between 10°C and 50°C.

2. The method according to claim 1, wherein
R¹ and R² represent hydrogen, chlorine, fluorine, bromine, iodine, CF₃, methyl, C₁-C₆-alkoxy, -OCF₃, -C(CH₃)₃, -CH(CH₃)₂,
R³ represents hydrogen, Cl, F, Br, C₁-C₆-alkyl, C₁-C₆-alkoxy, -C(CH₃)₃, and
X represents hydroxyl, F, Cl, Br, C₁-C₆-alkoxy.

3. The method according to claim 1, wherein
R¹ and R² represent hydrogen, chlorine, fluorine, bromine, iodine, CF₃, methyl, C₁-C₄-alkoxy, -C(CH₃)₃, -CH(CH₃)₂;
R³ represents hydrogen, Cl, F, Br, C₁-C₆-alkyl, C₁-C₄-alkoxy, -C(CH₃)₃; and
X represents hydroxyl, F, Cl, C₁-C₄-alkoxy.

4. A method according to claim 1 for preparing 1-(2,4-dihydroxyphenyl)-2-(4-methoxyphenyl)ethanone **characterized in that** 4-methoxyphenylacetic acid or 4-methoxyphenylacetyl chloride is reacted with resorcinol and with hydrogen fluoride, without further diluents.

5. The method according to claim 1, **characterized in that** the molar ratio of hydrogen fluoride to the phenol of the formula (III) is in the range of 50:1 to 10:1.

6. The method according to claim 1, **characterized in that** the reaction proceeds in the presence of a Lewis acid.

7. A method for preparing 1-(2,4-dihydroxyphenyl)-2-(4-methoxyphenyl)ethanone, **characterized in that** 4-methoxyphenylacetic acid or 4-methoxyphenylacetyl chloride is reacted with resorcinol and with hydrogen fluoride at a molar ratio of hydrogen fluoride to resorcinol in the range of 50:1 to 10:1 and at a temperature in the range of 0°C to 40°C, wherein no further diluent is used, and wherein the reaction is performed pressureless under autologous pressure.

8. A method for preparing compounds of the general formula (IV), 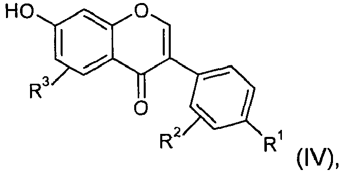 by converting the compounds of the general formulae (II) and (III) according to the method of claim 1 to compounds of the general formula (I) and then cyclizing the latter to flavones of the general formula (IV).

## Revendications

1. Procédé destiné à la production de 2,4-dihydroxy phényl benzyl cétones de formule (I), 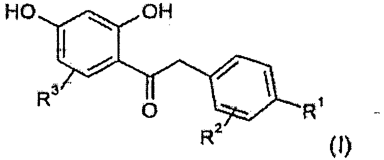
dans laquelle R¹ et R² correspondent à hydrogène, chlore, fluor, brome, iode, CF₃, méthyle, ainsi qu'alcoxy, OCF₃, C(CH₃)₃ et CH(CH₃)₂ éventuellement substitué;
R³ représente hydrogène, CI, F, Br, ainsi qu'alkyle éventuellement substitué, alcoxy éventuellement substitué et C(CH₃)₃ ; et
X représente hydroxy, F, CI, Br, ainsi qu'alcoxy éventuellement substitué,
par la mise en réaction de dérivés d'acide phénylacétique de formule (II) avec des phénols de la formule (III) dans du fluorure d'hydrogène, 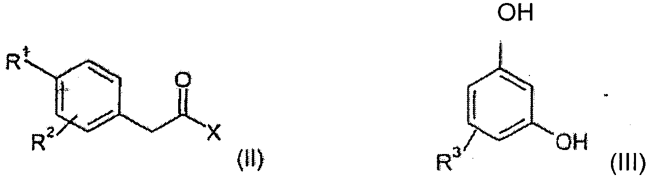 dans lequel aucun autre diluant n'est utilisé, dans lequel la réaction a lieu sans aucune pression sous l'effet de sa propre pression et dans lequel la température de réaction est comprise entre 10 °C et 50 °C.

2. Procédé selon la revendication 1, dans lequel
R¹ et R² correspondent à hydrogène, chlore, fluor, brome, iode, du CF₃, méthyle, alcoxy C₁-C₆, -OCF₃, C(CH₃)₃ et CH(CH₃)₂ ;
R³ représente hydrogène, CI, F, Br, alkyle C₁-C₆, alcoxy C₁-C₆, et C(CH₃)₃ ; et
X représente hydroxy, F, Cl, Br et alcoxy C₁-C₆.

3. Procédé selon la revendication 1, dans lequel
R¹ et R² correspondent à hydrogène, chlore, fluor, brome, iode, CF₃, méthyle, alcoxy C₁-C₄, C(CH₃)₃, et CH(CH₃)₂ ;
R³ représente hydrogène, Cl, F, Br, alkyle C₁-C₆, alcoxy C₁-C₄ et C(CH₃)₃ ; et
X représente hydroxy, F, CI et alcoxy C₁-C₄.

4. Procédé selon la revendication 1 destiné à la production de 1-(2,4-dihydroxy phényl)-2-(4-méthoxyphényl) éthanone, **caractérisé en ce que** de l'acide 4-méthoxyphényl acétique ou du chlorure de 4-méthoxyphényl acétyle est mis à réagir avec du résorcinol et avec du fluorure d'hydrogène sans aucun autre diluant.

5. Procédé selon la revendication 1, **caractérisé en ce que** le rapport molaire du fluorure d'hydrogène sur le phénol de formule (III) se situe dans une plage comprise entre 50:1 et 10:1.

6. Procédé selon la revendication 1, **caractérisé en ce que** la réaction se déroule en présence d'un acide de Lewis.

7. Procédé destiné à la production de 1-(2,4-dihydroxy phényl)-2-(4-méthoxyphényl) éthanone, **caractérisé en ce que** de l'acide 4-méthoxyphényl acétique ou du chlorure de 4-méthoxyphényl acétyle est mis à réagir avec du résorcinol et avec du fluorure d'hydrogène, conformément à un rapport molaire du fluorure d'hydrogène sur le résorcinol se situant dans une plage comprise entre 50:1 et 10:1 et à une température se situant dans une plage comprise entre 0 °C et 40 °C, dans lequel aucun autre diluant n'est utilisé et dans lequel la réaction a lieu sans aucune pression sous l'effet de sa propre pression.

8. Procédé destiné à la production de liaisons de formule générale (IV), selon lequel les liaisons des formules générales (II) et (III) sont transformées en liaisons de formule générale (I) conformément au procédé selon la revendication 1 et celles-ci sont ensuite cyclisées en flavones de formule générale (IV)
